# EUROPEAN PATENT APPLICATION

(11) **EP 1 967 226 A1**
(43) Date of publication of application: **10.09.2008**
(21) Application number: 07103818.6
(22) Date of filing: 09.03.2007
(51) Int. Cl.: A61N 1/34, A61N 1/36

(54) **Audio-modulated transcutaneous electrical nerve stimulation**

(71) Applicant: Koninklijke Philips Electronics N.V., 5621 BA Eindhoven (NL)
(72) Inventor: The designation of the inventor has not yet been filed
(74) Representative: Schoenmaker, Maarten

(57) **Abstract**

An apparatus for electrically stimulating living tissue of a person, comprises a first pulse generator for generating a first electrical pulse signal and at least one pair of electrodes, adapted to be applied to the skin of said person. The apparatus further comprises connecting means for connecting the pulse generator with the electrodes and for transferring the first pulse signal from the first pulse generator to the electrodes. The apparatus further comprising a modulator arranged between the first pulse generator and the electrodes and being adapted for modulating the first pulse signal by a modulating signal. The modulator is adapted to modulate the first signal by a modulating signal perceivable by human beings.

## Description

### BACKGROUND OF THE INVENTION

Currently, Transcutaneous Electrical Nerve Stimulation (TENS) is a common used form of electroanalgesia (electrical pain relief). Clinical reports exist concerning the use of TENS for many types of conditions like low-back pain, myofascial and arthritic pain, sympathetically mediated pain, bladder incontinence, neurogenic pain, visceral pain and postsurgical pain.

TENS is the application of electrical stimulation of at the surface of the skin, primarily for pain relief. TENS is applied via external surface electrodes with some sort of electrical waveform characterized by frequency, pulse duration and amplitude.

Relieving pain by application of electricity is known a long time, but the it gained substantial interest after the publication of Melzack & Wall on the gate control theory in 1965, providing a scientific base on the mechanism of pain reduction. TENS is drug free, non invasive, non addictive and it has hardly any contra indications.

Nevertheless the technology is not used extensively for the treatment of pain for several reasons; one is patient discomfort like associated sensations during treatment of electrical stimulation. Patients who are not satisfied or comfortable with the treatments they receive are often not willing to maintain the prescribed treatment and a lack of compliance results in inefficient treatment response. Patient comfort is especially important in electrical stimulation treatment due to extensive periods the treatment is often given.

TENS is also applied in physiotherapy and in recovering or improving circadian rythms in Alzheimer patients. Additionally, TENS is used in a large number of commercial products, e.g. Birdy Body Trimmer, Abtronic, Ab Energizer, for such diverse purposes as fitness, massage, exercising muscles, body tuning/sculpting and losing weight.

All of the above mentioned applications for TENS suffer of feeling sensations not wanted by the users.

### SUMMARY OF THE INVENTION

Consequently the present invention provides an apparatus for electrically stimulating living tissue of a person, the apparatus comprising a first pulse generator for generating a first electrical pulse signal, at least one pair of electrodes, adapted to be applied to the skin of said person, connecting means for connecting the pulse generator with the electrodes and for transferring the first pulse signal from the first pulse generator to the electrodes and a modulator arranged between the first pulse generator and the electrodes and being adapted for modulating the first pulse signal by a modulating signal, wherein the modulator is adapted to modulate the first signal by a modulating signal perceivable by human beings.

The invention also relates to a method for electrically stimulating living tissue of a person, wherein a first electrical pulse signal is applied to the skin of a person through electrodes applied on the skin of said person, wherein said pulse signal is modulated by a modulating signal before it is applied to the electrodes, wherein the modulating signal is a signal perceivable by a human being.

The signal thus applied to the electrodes and hence to the skin of the patient gives a sensation already perceived by the patient in a different way. This combined sensation masks the sensation of the first signal applied to the patient often perceived as uncomfortably by the patient.

It is noted that US-A-4 989 605 discloses an apparatus for TENS, wherein the modulation takes place. However the modulating signal is a signal not perceivable by the user.

According to a first embodiment the modulator is adapted to modulate the first signal with a game parameter signal appearing on an output of a game console. The output of the game console may be the acceleration of the person who's role the player is playing or the force said person experiences. As the player perceives already this signal by looking the game and playing it, the experience of the first signal is masked.

The invention also relates to a method as stated above, wherein the modulating signal is an output signal of a game console representing a game parameter.

It has appeared to the inventors that audio signals are in particular able to mask the unpleasant sensations caused by the application of the first signal. Hence, according to a preferred second embodiment, the modulator is adapted to modulate the first signal with an audio signal. The system thus devised is indicated by the acronym Audio Modulated TENS or AM-TENS.

The invention also relates to a method of the kind referred to above, wherein the modulating signal is an audio signal.

Also when the apparatus is used for muscle stimulation synchronized muscle contraction and music rhythms can increase the relaxation effects of the muscle stimulation. The synchronized muscle contraction and musical rhythms can increase the fitness performance. An example thereof is pace controlled running with music.

Further the appreciation of listening to certain types of music can be enhanced or intensified by the addition of a TENS signal that is audio modulated by the same music signal, in particular when the music contains many low frequency components.

It is emphasized that this embodiment is not only applicable for the aim mentioned beforehand, but also for improving speech intelligibility in adverse environmental conditions, like noise working conditions or noisy bars. Also speech intelligibility in hearing impaired persons can be greatly improved by combination of lip reading and the application of an AM-TENS signal audio modulated by the actual speech signal.

Although not required per se for the application of the invention, it is preferred if the person in question perceives the modulating signal not only by through the electrodes but also though the normal hearing system. Consequently preferably the apparatus comprises loudspeakers connected to the extra source.

To avoid disturbance of people surrounding the person in question it is preferred if the loudspeakers are formed by earphones. Commonly, the extra source will be connected to the modulator by means of a wired connection. However, it is also conceivable that the extra source and the modulator are adapted for wireless communication with each other, which will increase the mobility of different components of the apparatus according to the invention. It is a simple matter to relocate the extra source and/or the modulator, and no additional cost of rewiring and excessive downtime is associated with such a move.

Modulation offers several possibilities; it has however appeared that the use of amplitude modulation leads to an attractive way of masking the unpleasant sensations of the first signal generally used for TENS.

In some applications it may however be desirable that the modulator is adapted to modulate the frequency of the first signal with the extra signal.

Often the first signal is a pulse signal. This offers the possibility to modulate the pulse width of this pulse signal.

Low frequency components of audio signals seem to have a more effective masking effect than high frequency components. The use of a filter enhances the presence of the low frequency components of the sound signal so that the thus filtered music signal has a more effective masking effect. It is however also possible to use an audio source adapted to generate an music signal with a high content of low frequency components. Examples thereof are music sources playing rock, house or organ music.

The apparatus is simplified if use is made of readily available audio sources, like sources of recorded music, like a CD-player or an MP3-player.

Especially for persons with a impaired hearing it is attractive if the audio source is formed by a microphone. Then the perception of sound is not only transferred through the impaired ear system but also through the electrodes enhancing the impaired perception.

Contrary to the use of a game console mentioned earlier, the game console can also be used as a source of sound, a principally alternative way of enhancing the perception of playing a game.

According to another preferred embodiment, the apparatus comprises at least a second pair of electrodes connected to a second pulse generator, wherein the second pulse generator is adapted to generate a second pulse signal and wherein the frequency of the second pulse signal differs little from the frequency of the first pulse signal. This embodiment makes use of fact that the human skin has a higher conductivity for high frequency signals than for low frequency signals. By transferring both high frequency signals through the skin and combining them the perception of the resulting signal with a frequency equal to the frequency difference of the high frequency signals is obtained.

Herein the word 'little' is understood to mean about 10%, meaning that the differential frequency is about one order of magnitude smaller than the frequencies of the pulse signals. The first pair of electrodes and/or the second pair of electrodes may be integrated in a pad adapted to be applied to the human skin. Multiple pairs of electrodes or multiple pads each pad comprising at least one pair of electrodes may be applied simultaneously to several parts of a human body or to parts of multiple human bodies simultaneously.

### BRIEF DESCRIPTION OF THE DRAWINGS

Subsequently the present invention will be elucidated with the help of the following drawings, wherein:
Fig. 1 shows a diagram of a TENS apparatus according to the prior art;
Fig. 2 shows a diagram of a first embodiment of a TENS apparatus according to the invention;
Fig. 3 shows a diagram of a second embodiment of the present invention;
Fig. 4 shows a part of a human body onto which the apparatus according to Fig. 3 is applied; and
Fig. 5 shows a diagram of a second embodiment of the present invention.

### DETAILED DESCRIPTION OF PREFERRED EMBODIMENTS

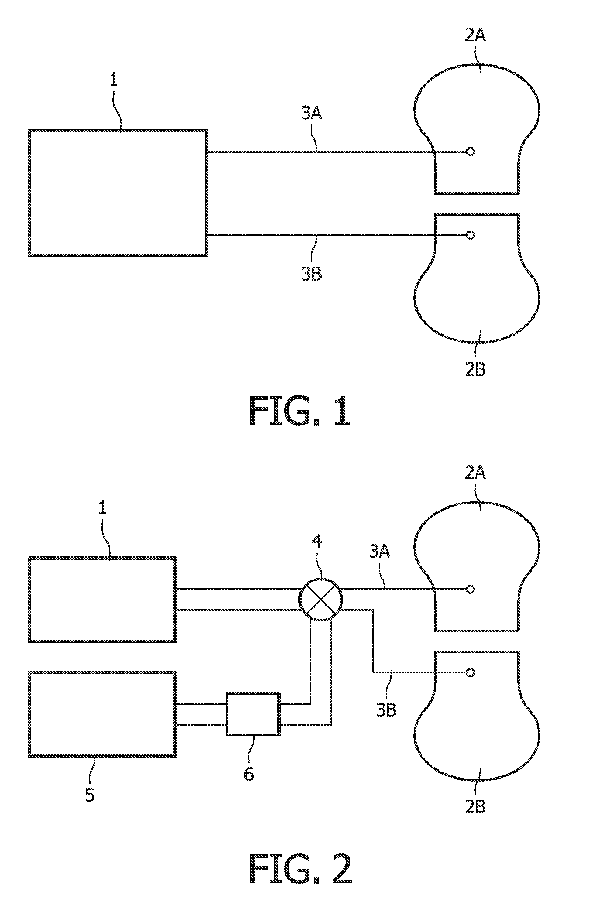

Figure 1 shows a diagram of a TENS apparatus according to the prior art. The apparatus comprises a generator 1 connected to two electrodes 2a and 2b adapted to be adhered or other wise connected to a part of the skin of the human body. Both electrodes 2a, 2b are connected to the generator by connecting cables 3, 3b respectively. The generator is adapted to generate a signal which is used for pain relief or muscle stimulation.

According to the invention the TENS apparatus comprises a modulator 4, which can be adapted to modulate the amplitude, the frequency, the duty cycle or another entity of the signal generated by the generator 1 (see Figure 2). The modulating signal is generated by a modulating signal generator 5, which can be formed by an audio source, like a CD-player or an MP3-player. Other sources of audio signals, like microphones, game consoles, TVs, etc. are not excluded. Further the modulating signal does not have to be an audio signal; rather it may be formed by a signal coming from another source like a signal from a game console representing a speed or a force. Herein it is important that the signal represents an entity perceivable by humans. Besides it is noted that the audio signal mentioned before may come from a game console as well. The audio source, the modulator 4, and the modulating signal generator 5 may be adapted for wireless communication with each other.

The modulating signal from the modulating signal generator is subjected to low pass filtering by a low pass filter 6, to remove high frequency components form the modulating signal. If the source itself is adapted to generate signals with emphasis on low frequencies the low pass filter may be left out.

In use the electrodes are adhered to the skin of the body on a mutual distance so that the electrical fields applied by the signals generated by the generator 1 lead to electrical fields at those parts of the human body wherein the effect aimed for is obtained. Herein the often unpleasant sensations of these fields are masked by the sensations of the fields generated by the modulating signal. The electrodes may be integrated with a pad to be adhered to the human skin to facilitate adhesion of the electrodes to the human skin.

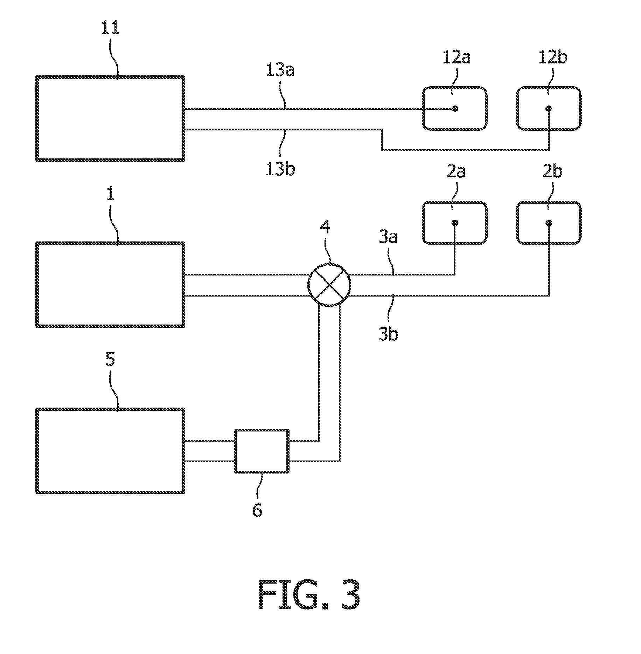

In Figure 3 an alternative embodiment is depicted wherein two additional electrodes 12a, 12b, together with an extra generator 11 are present. The electrodes 12a, 12b are connected to the generator 11 through connecting cables 13a, 13b. Both generators are adapted to generate a signal with a frequency about one order of magnitude higher than the frequency of the preceding embodiment, but wherein the difference between the frequencies is substantially equal to the frequency in the preceding embodiment. This embodiment makes use of the fact that the conductivity of the human skin is increases with frequency of the signal. The difference of the frequencies is the frequency of the perceived signal. It is noted that only one of the signals has to modulated by the modulating signal.

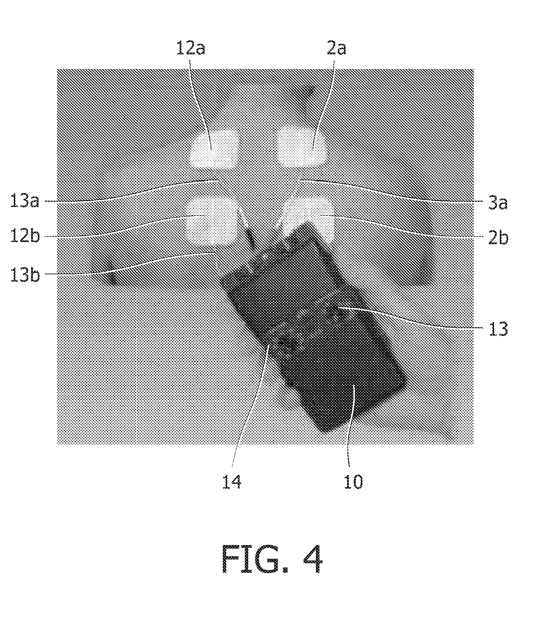

Figure 4 shows how the apparatus is applied to a human body. This figure shows a housing 10 in which the generators 1, 11, the generator 5 for the modulating signal, the low pass filter 6 and the modulator 4 are incorporated. The housing 10 further comprises control knobs 13, 14 for controlling frequency or other parameters of the signals. The electrodes 11a, 11b, 12a, 12b are applied to the skin of the human being after which the generators are witched on. The generators 1, 11 generate a pulse signal with a frequency having a small difference. The pulse signal generated by the pulse generator 1 is modulated in the modulator by the signal coming from the modulating generator 5, which is adapted to generate a signal which is perceivable by a human being, like an audio signal. The signal thus modulated is applied to the electrodes. The human being perceives the audio signal, which masks the effects of the pulse signals, adapted to counteract pain or to stimulate.

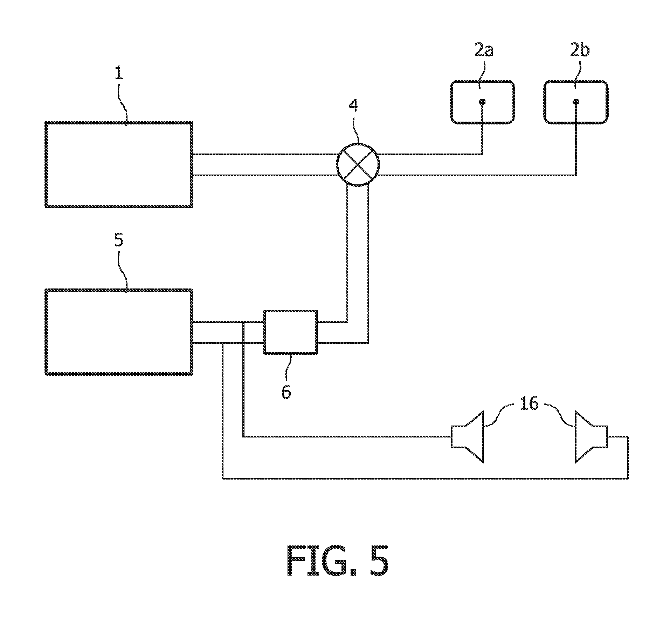

Finally Figure 5 shows a diagram of a apparatus depicted in Figure 2, but wherein the modulating signal generated by the modulating signal generator is also applied to a pair of loudspeakers 16. These loudspeakers are preferably formed by the loudspeakers of small ear phones.

## Claims

1. Apparatus for electrically stimulating living tissue of a person, comprising:
- a first pulse generator for generating a first electrical pulse signal;
- at least one pair of electrodes, adapted to be applied to the skin of said person;
- connecting means for connecting the pulse generator with the electrodes and for transferring the first pulse signal from the first pulse generator to the electrodes; and
- a modulator arranged between the first pulse generator and the electrodes and being adapted for modulating the first pulse signal by a modulating signal,
wherein the modulator is adapted to modulate the first signal by a modulating signal perceivable by human beings.

2. Apparatus as claimed in claim 1, wherein the modulator is adapted to modulate the first signal by a modulating signal being a game parameter signal appearing on an output of a game console.

3. Apparatus as claimed in claim 1, wherein the modulator is adapted to modulate the first signal by a modulating signal being an audio signal.

4. Apparatus as claimed in claim 3, **characterized by** an audio source adapted to generate the audio signal.

5. Apparatus as claimed in claim 4, **characterized by** loudspeakers connected to the source of the audio signal.

6. Apparatus as claimed in claim 5, **characterized in that** the loudspeakers are formed by earphones.

7. Apparatus as claimed in any of the preceding claims, wherein the modulator is adapted to modulate the amplitude of the first signal with the extra signal.

8. Apparatus as claimed in any of the preceding claims, wherein the modulator is adapted to modulate the frequency of the first signal with the extra signal.

9. Apparatus as claimed in any of the preceding claims, wherein the modulator is adapted to modulate the pulse width of the first signal with the extra signal.

10. Apparatus as claimed in any of the preceding claims, wherein a low pass filter is arranged between the extra source and the modulator.

11. Apparatus as claimed in any of the claims 4-10, wherein the audio source is a source of recorded music, like a CD-player or an MP3-player, and/or a source of live music.

12. Apparatus as claimed in any of the claims 4-10, wherein the audio source is a microphone.

13. Apparatus as claimed in any of the claims 4-10, wherein the audio source is an audio output of a game console.

14. Apparatus as claimed in any of the claims 4-10, wherein the audio source as adapted to generate an music signal with a high content of low frequency components.

15. Apparatus as claimed in any of the preceding claims, comprising at least a second pair of electrodes connected to a second pulse generator, wherein the second pulse generator is adapted to generate a second pulse signal and wherein the frequency of the second pulse signal differs little from the frequency of the first pulse signal.

16. Method for electrically stimulating living tissue of a person, wherein a first electrical pulse signal is applied to the skin of a person through electrodes applied on the skin of said person, wherein said pulse signal is modulated by a modulating signal before it is applied to the electrodes, wherein the modulating signal is a signal perceivable by a human being.

17. Method as claimed in claim 16, wherein the modulating signal is an output signal of a game console representing a game parameter.

18. Method as claimed in claim 16, wherein the modulating signal is an audio signal.
